# EUROPÄISCHE PATENTSCHRIFT

(11) **EP 0 633 037 B1**
(45) Veröffentlichungstag und Bekanntmachung des Hinweises auf die Patenterteilung: **18.02.1998**
(21) Anmeldenummer: 94106166.5
(22) Anmeldetag: 21.04.1994
(51) Int. Cl.: A61M 39/00

(54) **Konnektorsystem zum Verbinden von Flüssigkeitsbehältern**
Connection system for the joining of fluid containers
Système de raccord pour connecter des récipients de fluide

(30) Priorität: 26.04.1993 DE 4313636
(43) Veröffentlichungstag der Anmeldung: 11.01.1995
(73) Patentinhaber: B. BRAUN MELSUNGEN AG, 34212 Melsungen (DE)
(72) Erfinder: Wesseler, Matthias, D-49326 Melle (DE)
(74) Vertreter: Selting, Günther, Dipl.-Ing.

(56) Entgegenhaltungen:
- EP-A- 0 511 538
- DE-A- 3 618 739
- US-A- 5 113 571

## Beschreibung

Die Erfindung betrifft ein Konnektorsystem zum Verbinden von Flüssigkeitsbehältern, insbesondere von Behältern mit medizinischen Flüssigkeiten.

In der Medizintechnik, jedoch auch auf anderen Gebieten wie z. B. im Laborbereich, ist es oftmals erforderlich, Flüssigkeiten in einer bestimmten Reihenfolge von einem Behälter in einen anderen zu überführen und sie nach einer bestimmten Zeit oder einem vorgegebenen Ablauf, etwa einem Mischen oder einer chemischen Reaktion, weiterzuleiten.

Als Beispiel sei die Dialyse nach dem Hämofiltrationsverfahren genannt. Bei diesem Verfahren kommt eine Bicarbonat-Elektrolytlösung als Substituat zur Anwendung.

Da die beiden Einzellösungen, Bicarbonatlösung und Elektrolytlösung, in vermischtem Zustand nur etwa 24 Stunden haltbar sind, werden sie in getrennten Beuteln gelagert und erst kurz vor der Anwendung miteinander vermischt. Da die Übertragung nur einer der beiden Komponenten auf den Patienten kritisch sein kann, ist dafür Vorsorge zu treffen, daß vor dem Anschließen an ein Überleitungsset auf jeden Fall die beiden Lösungen miteinander vermischt werden und ein versehentlicher Anschluß nur einer Lösungskomponente durch effektive Vorkehrungen ausgeschlossen wird.

Bei dem vorstehend genannten Anwendungsgebiet ist es bekannt, die beiden Lösungen getrennt voneinander in zwei getrennten Beuteln aufzubewahren und erst unmittelbar vor der Anwendung miteinander zu vermischen. Dieses System schließt aber nicht aus, daß infolge unachtsamer Bedienung das Vereinigen und Mischen unterlassen wird und nur eine der Komponenten in das Überleitungsset abgegeben wird.

Ein derartiges System ist beispielsweise bekannt geworden aus der DE-36 27 231 A1, in welcher ein sogenanntes Überleitungsgerät zum Mischen von in unterschiedlichen Behältern befindlichen Medikamenten beschrieben ist. Diesem haftet jedoch eben jener weiter oben beschriebene Nachteil an. Zwar ist in dieser Druckschrift eine zeitliche Abfolge der Herstellung der Verbindung zwischen den beiden Behältern beschrieben. Dies jedoch nicht vor dem Hintergrund, daß die Verbindung in einer zwingend notwendigen zeitlichen Abfolge zu erfolgen hat.

Außerdem ist es bekannt, daß beide Lösungen durch eine Wand getrennt in einem Zweikammer-Beutel gelagert werden. Dadurch wird die zulässige Lagerzeit durch die Komponente mit der kürzeren Haltbarkeit bestimmt, so daß beim Überschreiten des Haltbarkeitsdatums auch die andere Komponente, die an sich noch ohne weiteres verwendbar wäre, verloren geht. Beim Zweikammer-Beutel sind ferner immer zwei Flüssigkeiten in bestimmter Menge und Konzentration einander zugeordnet, so daß für jede Kombination die geeigneten Paarungen auf Vorrat gehalten werden müssen.

Aus der EP-A-0511538 ist schließlich noch eine Ventilvorrichtung für einen Katheter bekannt, mit der ein Flüssigkeitsbehälter mit einem Katheteransatz verbunden werden kann. Mit dieser Ventilvorrichtung ist es jedoch nicht möglich, Flüssigkeiten in einer bestimmten Reihenfolge von einem Behälter in einen anderen zu überführen und sie anschließend weiterzuleiten, da diese Ventilvorrichtung nur den Anschluß eines Flüssigkeitsbehälters an einen Katheteransatz erlaubt und wegen der besonderen Ausgestaltung der Ventilvorrichtung als Rückschlagventil nur die Zuführung von Flüssigkeit aus dem angeschlossenen Behälter zu dem Katheter ermöglicht.

Vor diesem Hintergrund ist es nun die Aufgabe der vorliegenden Erfindung, ein Konnektorsystem zu schaffen, das konstruktiv bedingt nur eine einzige vorgegebene Verbindungsfolge von Flüssigkeitsbehältern ermöglicht und Fehlbedienungen ausschließt. Das System soll es ferner ermöglichen, Flüssigkeitsbehälter miteinander zu verbinden, die unabhängig voneinander gefüllt und gelagert sind.

Die Lösung der Aufgabe erfolgt durch die Merkmale des Anspruchs 1. Zweckmäßige Ausgestaltungen sind Gegenstand der Unteransprüche.

Das erfindungsgemäße Konnektorsystem besteht - wie das System gemäß der DE-A-36 27 231 - aus zwei Verbindungsteilen, von denen jedes an je einen Flüssigkeitsbehälter anschließbar ist. Die Verbindungsteile sind erfindungsgemäß miteinander allerdings nur unter Verwendung eines Zwischenteils verbindbar, so daß auf diese Weise die Flüssigkeit von dem einen in den anderen Behälter übergeleitet werden kann, wobei die beiden Flüssigkeiten vermischt werden oder ggf. miteinander reagieren. Dabei ist es wesentlich, daß das Zwischenteil vor Herstellung der Verbindung mit dem zweiten Verbindungsteil im Inneren des ersten Verbindungsteils sitzt.

Erfindungsgemäß ist das besagte Zwischenteil vorgesehen, welches erst ein Verbinden der beiden Verbindungsteile miteinander ermöglicht. Dieses Zwischenteil ermöglicht desweiteren einen Anschluß des einen Behälters an einen weiteren Behälter oder an eine Leitung, und zwar nach Entfernung des einen Verbindungsteils durch Ankoppeln eines Anschlußstückes des weiteren Behälters oder der weiteren Leitung. Dazu ist das zwischenteil mit dem ersten Verbindungsteil lösbar, mit dem zweiten unlösbar verbunden. Damit ist als Verbindungsfolge zwingend vorgegeben, daß zunächst die Flüssigkeit aus dem ersten Behälter in den zweiten Behälter übergeleitet wird, daß dann das Zwischenteil von dem ersten Behälter gelöst wird, mit dem zweiten jedoch verbunden bleibt, und daß dann erst die Flüssigkeit aus dem zweiten Behälter in einen weiteren Behälter oder eine Leitung überführt werden kann.

Das erste Verbindungsteil ist beispielsweise als üblicher männlicher Luerlockkonnektor ausgebildet. Das Zwischenteil ist dabei anfangs im Inneren des ersten Verbindungsteils angeordnet. Dadurch ist eine Konnektion des ersten Verbindungsteils an ein übliches weibliches Luerlockteil verhindert. Lediglich eine Konnektion mit dem zweiten Verbindungsteil des erfindungsgemäßen Konnektionssystems ist möglich.

Nach der Diskonnektion vom ersten Verbindungsteil und dem Zwischenteil ist das Zwischenteil im Inneren des zweiten Verbindungsteils angeordnet, wodurch erst eine Konnektion des zweiten Verbindungsteils mit einem üblichen männlichen Luerlockteil eines weiteren Beutels oder weiteren Leitung, bspw. eines Überleitungsets ermöglicht wird.

Das zwischenteil ist dabei mit einem Rückschlagventil ausgestattet, das ein Rückströmen der Flüssigkeit in den ersten Behälter verhindert, wenn diese nach Herstellung der Verbindung durch das Konnektorsystem vom ersten Behälter zur Vermischung mit der Flüssigkeit im zweiten Behälter fließt. Ein Rückschlagventil bei der Verbindung von zwei Teilen zur Anwendung im medizinischen Bereich ist beispielsweise bekannt aus der EP 0 257 880 A1. Jedoch findet sich in dieser Druckschrift nichts darüber hinausgehendes im Hinblick auf eine eindeutige zeitliche Abfolge der Konnektierung, wobei vorliegend die zeitliche Abfolge erst durch das Zwischenteil festgelegt wird.

Soll nun das Flüssigkeitsgemisch aus dem zweiten Behälter appliziert werden, so wird das zwischenteil an der Stelle der erwähnten lösbaren Verbindung zum ersten Verbindungsteil abgekoppelt und dann eine Verbindung beispielsweise mit einem Schlauchsystem hergestellt. Dabei erfolgt die Verbindung unter Aufhebung der Sperrwirkung des Rückschlagventils im Zwischenteil, d. h. Flüssigkeit kann nun vom zweiten, das zu applizierende Flüssigkeitsgemisch enthaltenden Behälter in umgekehrter Richtung durch das Zwischenteil fließen zu einer angeschlossenen Leitung, die beispielsweise zu einem Überleitungsset führt.

Weitere Merkmale und Vorteile der Erfindung ergeben sich aus der nachfolgenden Beschreibung eines Ausführungsbeispiels anhand der Zeichnung. In dieser zeigen:
- Fig. 1: das erste Verbindungsteil im Längsschnitt,
- Fig. 2: das Zwischenteil im Längsschnitt,
- Fig. 3: das zweite Verbindungsteil im Längsschnitt,
- Fig. 4: das erste Verbindungsteil mit daran angeschlossenem Zwischenteil,
- Fig. 5: das erste Verbindungsteil, das Zwischenteil und das zweite Verbindungsteil im miteinander verbundenen Zustand, und
- Fig. 6: das zweite Verbindungsteil mit daran angeschlossenem Zwischenteil und einem Konnektor des Anschlußsystems.

Das nachfolgend erläuterte Ausführungsbeispiel der Erfindung ist ein System zum Vermischen zweier Lösungen für ein Hämofiltrationsverfahren und zum Anschließen der Mischung an ein Überleitungsset. In den Figuren sind gleiche Teile stets mit denselben Bezugszeichen versehen.

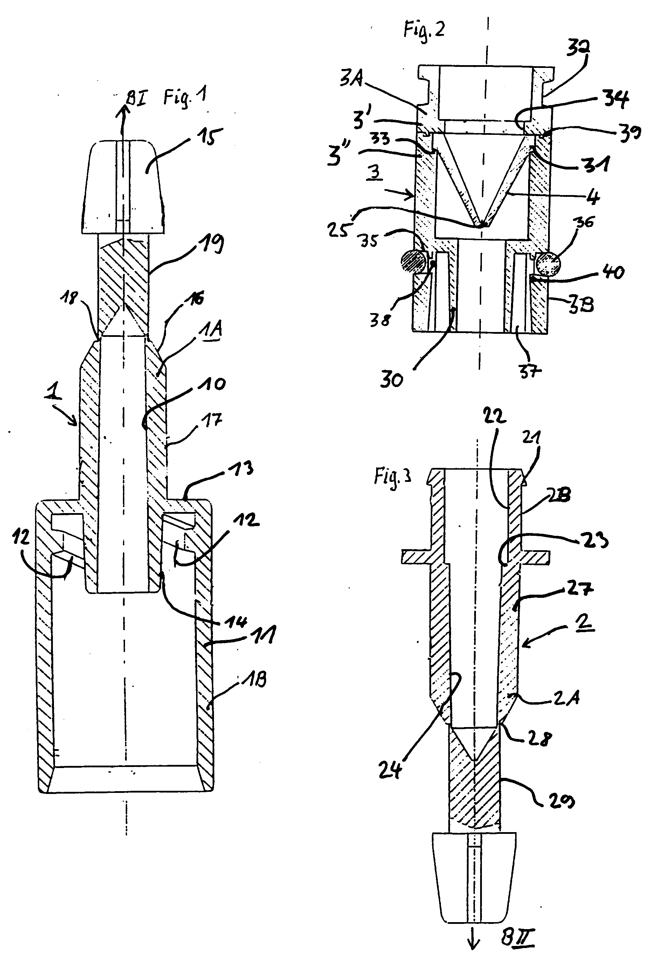

In den Fig. 1 bis 3 sind die drei Teile gezeigt, aus denen das erfindugsgemäße Konnektorsystem besteht, nämlich ein erstes Verbindungsteil 1, ein zweites Verbindungsteil 2 und ein Zwischenteil 3. Das erste Verbindungsteil 1 weist einen ersten Anschluß 1A und einen zweiten Anschluß 1B auf; in gleicher Weise besitzt das zweite Verbindungsteil 2 einen ersten Anschluß 2A und einen zweiten Anschluß 2B. Die beiden Anschlüsse 1A und 2A dienen zum Anschluß des Verbindungsteils an nicht dargestellte beutelförmige Behälter bei BI und BII, von denen der eine eine Bicarbonatlösung und der andere eine Elektrolytlösung enthält.

Der Anschluß 1A ist als hohlzylinderförmiger Körper oder Rohrstück 17 ausgebildet, dessen erstes Ende als Abbrechteil 19 entlang einer Sollbruchstelle 18 verschlossen ist. Das Abbrechteil 19 weist an seinem anderen Ende eine Anzahl von Flügeln 15 auf, die dazu bestimmt sind, daß sie beim Überstülpen eines Schlauches auf das Rohrstück 17 mit der Schlauchinnenwand in Reibkontakt kommen, wobei dann das abgebrochene Teil nicht auf das Rohrstück 17 zurückrutschen soll.

Der hohlzylinderförmige Körper oder das Rohrstück 17 selbst weist an dem der Sollbruchstelle 18 gegenüberliegenden Ende die Außenkontur eines Konus 14 auf, der in eine am Rohrstück 17 angeformte Außenhülse 11 hineinragt und von dieser umschlossen ist. Die Außenhülse 11 selbst ist hohlzylindrisch ausgebildet und weist in dem Bereich, der dem Konus 14 des Rohrstücks 17 gegenüberliegt, eine Schraubeinrichtung in Form von nach innen vorstehender kurzer Gewindegänge 12 auf. Das eine Ende der Außenhülse 11 ist mittels einer Ringplatte 13 verschlossen, die vom hohlzylindrischen Körper 17 durchsetzt ist, während das andere offene Ende den zweiten Anschluß 1B bildet.

Im Gebrauch wird ein nicht gezeigter Schlauch, der zu einem nicht gezeigten Beutel bei BI mit einer ersten Lösung führt, über das Rohrstück 17 bis zur Ringplatte 13 der Außenhülse 11 hin aufgeschoben.

Die Anschlüsse 1A und 1B liegen somit koaxial zueinander und sind durch einen durchgehenden Kanal 10 miteinander verbunden.

Das in Fig. 2 dargestellte zwischenteil 3 hat zwei Anschlüsse 3A und 3B. Der Anschluß 3A paßt mit seiner generell zylindrischen Form in die Außenhülse 11 des Anschlusses 1B des ersten Verbindungsteils 1 und weist an seinem Ende ein Außengewinde 32 auf, das mit den Gewindegängen 12 des Anschlusses 1B in Eingriff kommt.

Das Zwischenteil 3 ist im wesentlichen aus zwei hohlzylindrischen Teilstücken 3', 3'' zusammengesetzt, wobei der Außendurchmesser der beiden Teilstücke 3', 3'' etwas geringer ist als der Innendurchmesser der Außenhülse 11 des ersten Verbindungsteils 1, so daß das zwischenteil 3 - wie erwähnt - in diese Außenhülse 11 eingeschoben werden kann.

Die Länge des Zwischenteils 3 ist dabei so bemessen, daß es nach Einschieben und Herstellung der Schraubverbindung in der Außenhülse 11 im wesentlichen bündig mit dieser an dem Anschluß 1B abschließt.

An dem dem Einführende gegenüberliegenden Ende des ersten Teilstückes 3' ist eine ringartige Verengung 34 vorgesehen, die als Stützlager für ein hohlkegelartiges Rückschlagventil 4 dient. Dieses Rückschlagventil 4 weist an seinem Fuß einen ringförmigen Flansch 31 auf, von dem sich kegelartig verjüngend die Mantelflächen des Hohlkegels zur Spitze 25 erstrecken. Die Spitze 25 selbst ist mit einem Schlitz versehen, der sich auf Druck von der Seite des Hohlkegels her aufweitet. Das Material dieses Rückschlagventils 4 ist flexibel, und vorteilhafterweise Silikon.

An dem ersten Teilstück 3' des Zwischenteils 3 ist ein zweites Teilstück 3'' beispielsweise durch Verschweißen befestigt. Im Bereich der Verschweißnaht 39 ist in die Innenoberfläche des zweiten Teilstückes 3'' eine Ringnut 33 eingelassen, in welche der ringförmige Flansch 31 des Rückschlagventils 4 eingesetzt ist. Beim Zusammensetzen der beiden Teilstücke 3', 3'' wird dieser Ringflansch 31 axial zusammengepreßt und erhält somit einen festen Sitz. Hierdurch wird darüber hinaus eine hervorragende Dichtigkeit des Zwischenteils 3 erzielt. Der Durchmesser der Öffnung des Ringflansches 31 ist dabei so bemessen, daß er an einem weiter unten erläuterten rohrartigen Konnektionsstück eines weiteren Beutels oder eines Überleitungssystems dichtend zur Anlage kommt.

Die Spitze 25 des Rückschlagventils 4 ist dabei auf das dem Schraubgewinde 32 gegenüberliegende Ende des Zwischenteils 3 gerichtet, und läßt, sofern kein weiterer Konnektor eingesetzt ist, nur Flüssigkeit in diese Richtung durch, d. h. also vom Anschluß 3A zum Anschluß 3B.

Das zweite Teilstück 3'' des zwischenteils 3 weist weiterhin auf seiner Außenfläche eine ringartige Nut 35 auf, in welche eine Ringdichtung, vorteilhafterweise ein O-Ring 36, eingesetzt ist. Dieser O-Ring 36 kommt beim Konnektieren des Zwischenteils 3 mit dem ersten Verbindungsteil 1 mit der Innenoberfläche der Außenhülse 11 in dichtende Anlage und dichtet somit die gesamte erste Konnektionsanordnung ab.

Etwa in Höhe der Ringnut 35 sind in die Innenoberfläche des hohlzylindrischen zweiten Teilstücks 3'' zwei gegenüberliegende Öffnungen 38 eingelassen, die über sich axial von der Einführöffnung dieses zweiten Teilstücks 3'' zu diesen Öffnungen 38 erstreckende Nuten 37 verbunden sind.

Beabstandet von der Einführöffnung ist innerhalb des zweiten Teilstückes 3'' konzentrisch ein Rohrstück 30 mit geringem Wanddurchmesser angeordnet, das oberhalb der beiden Öffnungen 38 mit der Innenwand des zweiten Teilstückes 3'' verbunden ist. Dabei ist der Außendurchmesser dieses Rohrstückes 30 so bemessen, daß er in die erweiterte Einführungsöffnung 22 des zweiten Verbindungsteils 2 eingeführt werden kann, wie weiter unten noch näher erläutert werden wird.

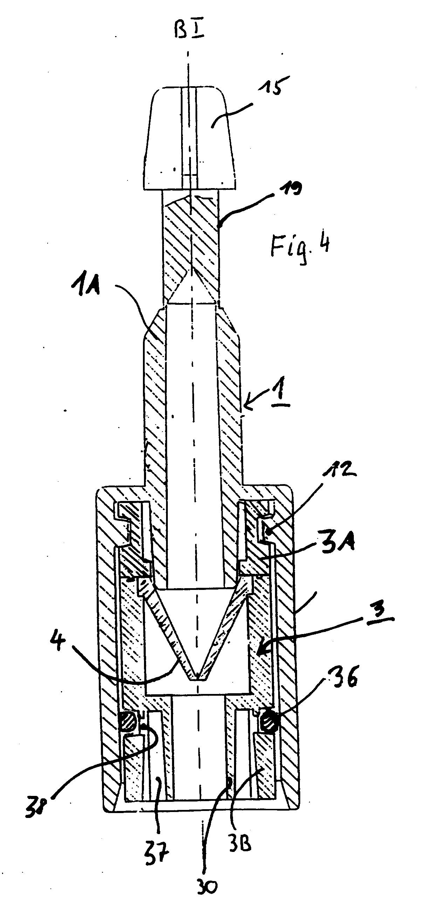

Fig. 4 zeigt sehr anschaulich das Verbindungsteil 1 mit darin eingesetztem Zwischenteil 3. Mit den derartig zusammengefügten Teilen 1 und 3 ist das Konnektorsystem einsatzbereit zur Verbindung mit dem Verbindungsteil 2, welches in Fig. 3 dargestellt ist.

Das zweite Verbindungsteil 2 weist wie das erste Verbindungsteil 1 einen hohlzylindrischen Körper oder Rohrstück 27 auf, das auf der einen Seite mit einem Abbrechteil 29 entlang einer Sollbruchstelle 28 verbunden ist, welches in ähnlicher Weise wie das Abbrechteil 19 des ersten Verbindungsteils 1 ausgebildet ist. Der dem Abbrechteil 29 zugewandte Abschnitt des Rohrstückes 27 bildet den Anschluß 2A des Verbindungsteils 2.

Der gegenüberliegende Anschluß 2B weist entlang seines Außenumfangs zwei gegenüberliegende Nasen 21 auf, welche durch in die weiter oben beschriebenen axialen Nuten 37 des Zwischenteils 3 passen. Der Strömungskanal 22 des zweiten Verbindungsteils 2 ist an seinem Anschluß 2B erweitert und geht unter Bildung eines sich radial nach innen verengenden Absatzes 23 in einen durchgehenden Strömungskanal 24 konstanten Durchmessers über. Der erwähnte Absatz 23 ist aber so ausgebildet, daß er mit dem Rohrstück 30 des Zwischenteils 3 beim Konnektieren formschlüssig zusammenwirkt und dieses Rohrstück 30, das aufgrund seiner geringen Wandstärke radial verformbar ist, zusammendrückt. Beim Vorschieben des zweiten Verbindungsteils 2 im Zwischenteil 3 überfahren dann die Nasen 21 eine radiale Verengung 40 vor den beiden Öffnungen 38 und schnappen nach Überfahren dieser Verengungen 40 in die beiden Öffnungen 38 ein und arretieren somit das zweite Verbindungsteil 2 an dem Zwischenteil 3 dauerhaft und unlösbar.

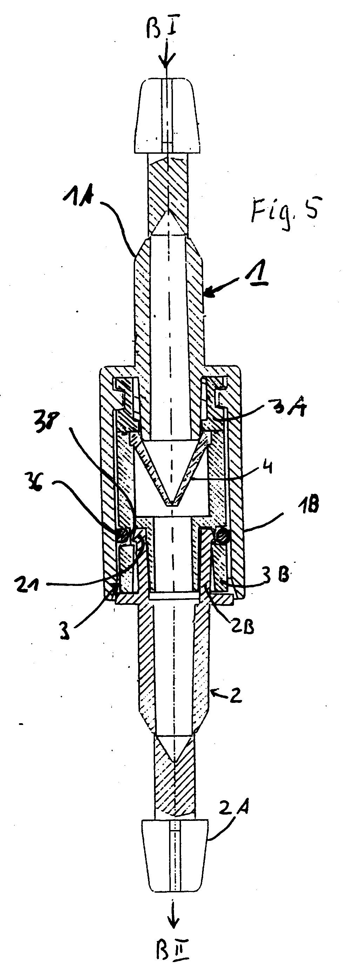

Aus dem unteren Teil von Figur 5 ist die eingerastete Verbindung zwischen dem Verbindungsteil 2 und dem Zwischenteil 3 ersichtlich.

Wie vorstehend bereits erläutert, wird zunächst das erste Verbindungsteil 1 mit dem Zwischenteil 3 verbunden, wobei dieses formschlüssig in das erste Verbindungsteil eingesetzt wird. Diese Anordung wird dann mit einer nicht dargestellten Kappe verschlossen und anschließend mit dem weiter oben erwähnten Schlauch zu einem ersten Beutel bei BI verbunden.

Das zweite Verbindungsteil 2 wird mit einer anderen Beutelanordnung bei BII verbunden.

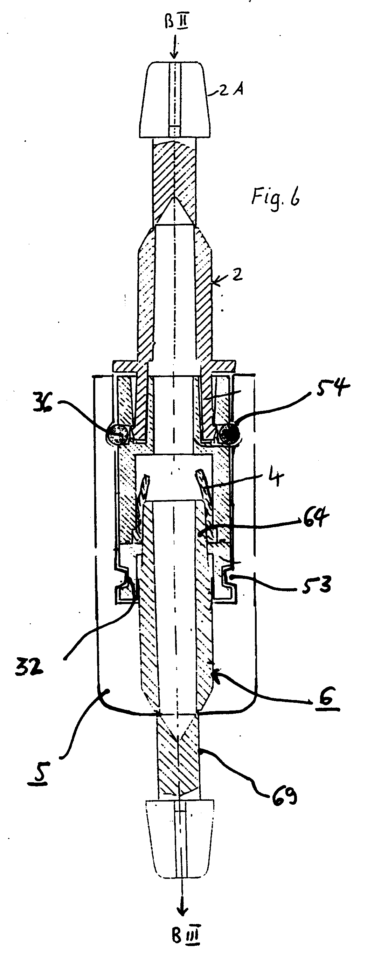

Um beide Beutelinhalte zu mischen, wird die erste Konnektionsanordnung aus erstem Verbindungsteil 1 und Zwischenteil 3 mit dem zweiten Verbindungsteil 2 verbunden. Anschließend werden beide Abbrechteile 19 bzw. 29 abgebrochen, so daß ein durchgehender Strömungskanal von dem einem Beutel bei BI zum anderen Beutel bei BII entsteht, der lediglich durch das Rückschlagventil 4 verschlossen ist, welches nur eine Strömung vom ersten Verbindungsteil 1 zum zweiten Verbindungsteil 2 hin zuläßt. Sind dann beide Beutelinhalte miteinander vermischt, so kann das erste Verbindungsteil 1 vom Zwischenteil 3 abgeschraubt werden, während das Zwischenteil 3 aufgrund seiner kraft- und formschlüssigen Verbindung am zweiten Verbindungsteil 2 verbleibt. In die nunmehr gebildete zweite Konnektionsanordnung aus dem zweiten Verbindungsteil 2 mit dem Zwischenteil 3 kann ein drittes Verbindungsteil 6 (Fig. 6) mit einem Rohrstück 64 eingesetzt werden, das so bemessen ist, daß es den Hohlkegel des Rückschlageventils 4 unter Öffnen des Ventils durchsetzt. Danach kann der Beutelinhalt bestimmungsgemäß aus dem zweiten Beutel überführt werden.

Aus Fig. 6 ist im übrigen eine Hülse 5 ersichtlich, die nach Art einer Überwurfhülse das Verbindungsteil 6 übergreift. Die Hülse 5 ist im Inneren so ausgebildet, daß dort Gewindegänge 53 vorgesehen sind, welche mit dem schon beschriebenen Schraubgewinde 32 des ersten Teilstücks 3' des Zwischenteils 3 verschraubbar sind.

Die Hülse 5 ist so dimensioniert, daß sie die zweite Konnektionsanordnung aus dem zweiten Verbindungsteil 2 mit dem Zwischenteil 3 soweit übergreift, daß sie mit ihrer Wandung zumindest den O-Ring 36 des Zwischenteils 3 übergreift, wenn die Hülse 5 mit dem Zwischenteil 3 verschraubt ist. Auf der Höhe des dann dichtend an der Innenoberfläche der Hülse 5 anliegenden O-Rings 36 ist eine ringförmige Nut 54 in die Innenoberfläche der Hülse 5 eingelassen, in welche der O-Ring 36 zu liegen kommt. Auf diese Weise dichtet die Hülse 5 die zweite Konnektionsanordnung vollständig ab.

## Patentansprüche

1. Konnektorsystem zum Verbinden von Flüssigkeitsbehältern, insbesondere mit medizinischen Flüssigkeiten, sowie eines der Flüssigkeitsbehälter mit einem Überleitungsset oder einem weiteren Behälter, wobei das Konnektorsystem zum Einhalten einer vorgegebenen Verbindungsfolge ein erstes und ein zweites Verbindungsteil (1, 2) mit jeweils einem ersten Anschluß (1A, 2A), der zum Verbinden mit dem Anschluß eines bestimmten Flüssigkeitsbehälters ausgebildet ist, und jeweils einem zweiten Anschluß (1B, 2B) aufweist,
sowie
ein Zwischenteil (3) mit zwei miteinander verbundenen Anschlüssen (3A, 3B) aufweist, von denen der erste (3A) zum lösbaren Verbinden mit dem zweiten Anschluß (1B) eines ersten Verbindungsteils (1) sowie mit einem Anschlußstück (6) eines Überleitungssets und der zweite (3B) zum unlösbaren Verbinden mit dem zweiten Anschluß (2B) des zweiten Verbindungsteils (2) ausgebildet ist, wobei das Zwischenteil (3) im Inneren des ersten Verbindungsteils (1) angeordnet werden kann und im wesentlichen bündig an seinem zweiten Anschluß (1B) abschließt.

2. Konnektorsystem nach Anspruch 1, dadurch gekennzeichnet, daß in dem Zwischenteil (3) ein Rückschlagventil (4) angeordnet ist, welches einen Flüssigkeitsstrom von dem ersten (3A) zu dem zweiten Anschluß (3B) des Zwischenteils (3) ermöglicht, in entgegengesetzter Richtung dagegen sperrt.

3. Konnektorsystem nach Anspruch 2, dadurch gekennzeichnet, daß das Rückschlagventil (4) beim Verbinden des ersten Anschlusses (3A) des Zwischenteils (3) und einem weiteren Behälter (BIII) oder einem Überleitungsset zwangsweise entgegen seiner Sperrwirkung geöffnet werden kann und einen Flüssigkeitsstrom von dem zweiten Anschluß (3B) zu dem ersten Anschluß (3A) ermöglicht.

4. Konnektorsystem nach einem der Ansprüche 1 bis 3, dadurch gekennzeichnet, daß die ersten und die zweiten Anschlüsse (1A, 2A, 3A, 1B, 2B, 3B) des ersten und/oder des zweiten Verbindungsteils (1, 2) und/oder des Zwischenteils (3) koaxial angeordnet sind.

5. Konnektorsystem nach einem der Ansprüche 1 bis 4, dadurch gekennzeichnet, daß die Verbindung zwischen dem zweiten Anschluß (1B) des ersten Verbindungsteils (1) und dem ersten Anschluß (3A) des Zwischenteils (3) als Schraubverbindung ausgebildet ist.

6. Konnektorsystem nach einem der Ansprüche 1 bis 5, dadurch gekennzeichnet, daß die Verbindung zwischen dem zweiten Anschluß (2B) des zweiten Verbindungsteils (2) und dem zweiten Anschluß (3B) des Zwischenteils (3) als Einschnappverbindung ausgebildet ist.

7. Konnektorsystem nach einem der Ansprüche 1 bis 6, dadurch gekennzeichnet, daß der erste Anschluß (3A) des Zwischenteils (3) an seiner Außenseite zum Verbinden mit dem zweiten Anschluß (1B) des ersten Verbindungsteils (1) und an seiner Innenseite als weiblicher Luer-Konus zum Verbinden mit einem weiteren Behälter (BIII) oder einem Leitungsanschluß mit männlichem Luer-Konus ausgebildet ist.

8. Konnektorsystem nach Anspruch 2 und 7, dadurch gekennzeichnet, daß das Rückschlagventil (4) des Zwischenteils (3) im Aufnahmebereich des männlichen Luer-Konus angeordnet ist und von diesem beim Anschließen eines weiteren Behälters (BIII) oder eines Leitungsanschlusses geöffnet wird.

## Claims

1. A connector system for connecting liquid containers, in particular such containers holding medical liquids, and for connecting one of said containers with a transfer set or another container, said connector system being provided with first and second connector means (1, 2) so as to maintain the predetermined connection sequence, each connector means having a first connector (1A, 2A) designed for connection with the connector of a particular liquid container, and a second connector (1B, 2B),
as well as
an intermediate member (3) with two interconnected connectors (3A, 3B), the first (3A) of which is adapted to be releasably connected to the second connector (1B) of a first connector (1B) of a first connector means (1) and to a connector member (6) of a transfer set and the second (3B) of which is adapted to be permanently connected to the second connector (2B) of the second connector means (2), the intermediate member (3) being adapted to be disposed inside the first connector means (1) and terminating substantially flush with the second connector (1B) thereof.

2. The connector system of claim 1, characterized in that a nonreturn valve (4) is arranged in said intermediate member (3), said valve allowing a liquid flow from said first (3A) to said second connector (3B) of said intermediate member (3), yet blocks the flow in the opposite direction.

3. The connector system of claim 2, characterized in that said nonreturn valve (4) may forcibly be opened against its blocking effect when connecting said first connector (3A) of said intermediate member (3) and a further container (BIII) or a transfer set, thereby allowing a liquid flow from said second connector (3B) to said first connector (3A).

4. The connector system of one of claims 1 to 3, characterized in that said first and second connectors (1A;2A, 3A, 1B, 2B, 3B) of said first and/or second connector means (1, 2) and/or said intermediate member (3) are disposed coaxially.

5. The connector system of one of claims 1 to 4, characterized in that the connection between said second connector (1B) of said first connector means (1) and said first connector (3A) of said intermediate member (3) is arranged as a threaded connection.

6. The connector system of one of claims 1 to 5, characterized in that the connection between said second connector (2B) of said second connector means (2) and said second connector (3B) of said intermediate member (3) is arranged as a snap-in connection.

7. The connector system of one of claims 1 to 6, characterized in that said first connector (3A) of said intermediate member (3) is provided on its outer surface for connection to said second connector (1B) of said first connector means (1) and has its inner surface designed as a female Luer cone for connection to a further container (BIII) or a conduit connector with a male Luer cone.

8. The connector system of claims 2 and 7, characterized in that said nonreturn valve (4) of said intermediate member (3) is disposed in the receiving portion of the male Luer cone and is opened by the same upon connection of a further container (BIII) or a conduit connector.

## Revendications

1. Système formant raccord servant à raccorder des récipients à fluides, contenant notamment des fluides médicaux, ainsi que l'un des réservoirs à liquides à un ensemble de canalisation de transition ou à un autre récipient, le système formant raccord comportant, pour le respect d'une séquence prédéterminée de liaison, des première et seconde parties de raccordement (1, 2) comportant des premiers raccords respectifs (1A, 2A) agencés de manière à établir la liaison avec le raccord d'un récipient à liquide déterminé, et des seconds raccords respectifs (1B, 2B), et comporte une partie intercalaire (3) possédant deux raccords (3A, 3B) reliés entre eux, parmi lesquels le premier raccord (3A) est agencé pour établir une liaison amovible avec le second raccord (1B) d'une première partie de raccordement (1) et avec un élément de raccordement (6) d'un seul ensemble de canalisation de transition et le second (3B) est agencé pour établir une liaison inamovible avec le second raccord (2B) de la seconde partie de raccordement (2), la partie intercalaire (3) pouvant être disposée à l'intérieur de la première partie de raccordement (1) et se terminant essentiellement de niveau sur son second raccord (1B).

2. Système formant raccord selon la revendication 1, caractérisé en ce que dans la partie intercalaire (3) est disposée une soupape antiretour (4), qui permet le passage d'un écoulement de liquide du premier raccord (3A) au second raccord (3B) de la partie intercalaire (3), alors qu'elle réalise un blocage dans la direction opposée.

3. Système formant raccord selon la revendication 2, caractérisé en ce que la soupape antiretour (4) lors de la liaison du premier raccord (3A) de la partie intercalaire (3) à un autre récipient (BIII) ou à un ensemble de canalisation de transition, peut être ouverte de façon impérative à l'encontre de son action de blocage, et permet un écoulement de liquide depuis le second raccord (3B) vers le premier raccord (3A).

4. Système formant raccord selon l'une des revendications 1 à 3, caractérisé en ce que les premiers et seconds raccords (1A, 2A, 3A, 1B, 2B, 3B) de la première partie de raccordement et/ou de la seconde partie de raccordement (1, 2) et/ou de la partie intercalaire (3) sont disposés coaxialement.

5. Système formant raccord selon l'une des revendications 1 à 4, caractérisé en ce que la liaison entre le second raccord (1B) de la première partie de raccordement (1) et le premier raccord (3A) de la partie intercalaire (3) est agencée sous la forme d'une liaison à vis.

6. Système formant raccord selon l'une des revendications 1 à 5, caractérisé en ce que la liaison entre le second raccord (2B) de la seconde partie de raccordement (2) et le second raccord (3B) de la partie intercalaire (3) est agencée sous la forme d'une liaison à encliquetage.

7. Système formant raccord selon l'une des revendications 1 à 6, caractérisé en ce que le premier raccord (3A) de la partie intercalaire (3) est agencé, au niveau de sa face extérieure, pour établir la liaison avec le second raccord (1B) de la première partie de raccordement (1) et, au niveau de sa face intérieure, sous la forme d'un cône LUER femelle pour l'établissement de la liaison avec un autre récipient (BIII) ou un raccord de canalisation possédant un cône LUER mâle.

8. Système formant raccord selon les revendications 2 et 7, caractérisé en ce que la soupape antiretour (4) de la partie intercalaire (3) est disposée dans la zone de réception du cône LUER mâle et est ouverte par ce dernier lors du raccordement d'un autre récipient (BIII) ou d'un raccord de canalisation.
